(19) Europäisches Patentamt
European Patent Office
Office européen des brevets

(11) Publication number: 0 169 250 B1

(12) **EUROPEAN PATENT SPECIFICATION**

(45) Date of publication of patent specification: 19.04.89

(51) Int. Cl.⁴: **D 21 H 3/08,** C 07 D 307/60

(21) Application number: 84108354.6

(22) Date of filing: 16.07.84

(54) **Paper sizing agent.**

(43) Date of publication of application:
29.01.86 Bulletin 86/05

(45) Publication of the grant of the patent:
19.04.89 Bulletin 89/16

(84) Designated Contracting States:
AT BE CH DE FR GB IT LI LU NL SE

(56) References cited:
US-A-4 040 900
US-A-4 302 283
US-A-4 431 826

(73) Proprietor: NIPPON PETROCHEMICALS COMPANY, LIMITED
3-1, Uchisaiwaichō 1-chome Chiyoda-ku
Tokyo (JP)

(72) Inventor: Sato, Atsushi
15-23, Yakumo 2-chome Meguro-ku
Tokyo (JP)
Inventor: Murai, Yoshikazu
28-10, Shinohara Kita 1-chome Kohoku-ku
Yokohama-shi Kanagawa-ken (JP)
Inventor: Goto, Masahito
22, Higashi Furuichiba Saiwai-ku
Kawasaki-shi Kanagawa-ken (JP)

(74) Representative: Schübel-Hopf, Ursula et al
Strehl Schübel-Hopf Groening Schulz
Patentanwälte Maximilianstrasse 54 Postfach 22 14 55
D-8000 München 22 (DE)

EP 0 169 250 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

(1) Field of the Invention

This invention relates to a paper sizing agent for use in paper making. More particularly, the invention relates to a novel internal paper sizing agent which is effectively employed in the neutral to alkaline range without using aluminum sulfate as a fixing agent.

(2) Description of the Prior Art

In the paper manufacturing industry, sizing agents that are prepared from natural rosins or modified rosins, especially, fortified rosin sizing agents that are prepared by saponifying maleic-modified rosins, are regarded as most preferable ones and they are widely used. These sizing agents are used together with aluminum sulfate and they are fixed to paper fibers under acidic conditions of pH 4.0 to 5.0. Owing to the acidic conditions in the use of these sizing agents, the following disadvantages are brought about. That is, paper making machinery suffers from corrosion, the strength and durability of obtained paper are lowered, and inexpensive alkaline fillers such as calcium carbonate cannot be used because the alkaline fillers are decomposed under an acidic condition. Therefore, it has been difficult to reduce much the production cost for paper making.

For this reason, neutral sizing agents which can be fixed to wood pulp without using aluminum sulfate, were looked for and some neutral sizing agents that produce excellent sizing effect in the range of neutral to alkali have been proposed. For example, a sizing agent which is prepared by dispersing alkylketene dimer in water in the presence of cationized starch is known. The sizing agent is, however, defective in that the cost is high and it takes much time to produce desired sizing effect.

Besides the above sizing agent, several neutral sizing agents of alkenyl succinic anhydride type have been proposed.

For example, in U.S. Patent No. 3,102,064, there is proposed a sizing agent that is defined by the following general formula:

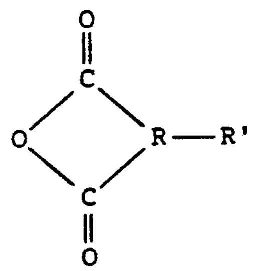

wherein R is a dimethylene group or a trimethylene group, and R' is a member selected from an alkyl group, alkenyl group, aralkyl group and aralkenyl group having more than 5 carbon atoms. The compounds exemplified are only branched olefins such as propylene oligomers and butene oligomers, or straight chain α-olefins. The sizing effect of all of these compounds are not satisfactory and they cannot be employed in practical paper making process.

In U.S. Patent No. 3,821,069, there is disclosed a sizing agent which comprises the reaction product between maleic anhydride and internal olefins and are represented by the following general formula:

$$R_x{-}CH_2{-}CH{=}CH{-}CH_2{-}R_y$$

wherein $R_x$ and $R_y$ are, respectively, alkyl radicals containing 4 to 10 carbon atoms.

In Japanese Preliminary Patent Publication No. 57—154495 is proposed a reactive sizing agent which consists of a mixture of alkenyl succinic anhydrides. The mixture is produced by adding maleic anhydride to a mixture of straight chain internal olefins that have 8 to 18 carbon atoms and double bonds thereof are almost evenly distributed at every position except α-position.

Furthermore, US—A—4 431 826 discloses a sizing agent which is a two component alkenyl succinic anhydride composition obtained by reacting maleic anhydride with $C_{13}$ to $C_{18}$ straight chain alpha-olefins, and $C_{14}$ to $C_{22}$ straight chain internal olefins.

The known sizing agents are, however not satisfactory in view of sizing effect. The internal olefins disclosed in the foregoing U.S. Patent No. 3,812,069 are obtained by disproportionation of α-olefins. The preparation of the internal olefins is difficult, which is disadvantageous because the production cost is high. The internal olefins that are disclosed in Japanese Preliminary Patent Publication No. 57—154495 are produced by a process in which straight chain paraffin is dehydrogenated at 400 to 600°C to obtain straight chain internal olefin and unreacted paraffin is then removed by adsorption with a molecular sieve, or by a process in which α-olefin of lower molecular ethylene polymer is subjected to isomerization and then to disproportionation. These processes are complicated and are high in cost, and the product is accordingly expensive.

Brief Summary of the Invention

It is, therefore, the primary object of the present invention to provide a novel and improved internal paper sizing agent that is free from the above drawbacks in the conventional art.

Another object of the invention is to provide a paper sizing agent which produces excellent sizing effect in neutral to alkaline regions without using aluminum sulfate as a fixing agent.

A further object of the invention is to provide a paper sizing agent which can be dispersed well in water, which is compatible with various kinds of paper making additives and which can be preserved for a long time.

Still a further object of the invention is to provide a paper sizing agent which can be produced without difficulties at a low cost.

Pursuant to the above objects, the inventors of the present application have carried out extensive investigations and experiments. As a result, the present invention has been accomplished.

According to the present invention, there is provided a paper sizing agent which comprises a reaction product which is obtained by adding maleic anhydride to a mixture of straight chain olefins having 14 to 36 carbon atoms, and/or the hydrogenation product of said reaction product which is characterized in that said mixture of straight chain olefins comprises 3 to 65 mole %, respectively, of olefins having a double bond at the 2nd, 3rd, 4th or 5th position and that the total quantity of these olefins is not less than 80 mole %.

Detailed Description of the Invention

The straight chain olefin starting materials for the preparation of neutral sizing agent of the present invention is characterized by the number of carbon atoms and the positions of double bonds in molecular chains.

If the number of carbon atoms is less than 14, satisfactory effects of a neutral sizing agent cannot be produced and the storage stability of sizing agent is low. Meanwhile, if the number of carbon atoms exceeds 36, the reaction product exerts sizing effect but the viscosity is too high which causes difficulties in practical uses. Incidentally, more preferable number of carbon atoms is 15 to 24. The number of carbon atoms of olefins may be the same or different from one another. In economical viewpoint, olefins of plurality kinds of carbon atom numbers may be preferably used.

According to the present invention, each quantity of a straight chain olefin having a double bond at 2nd, 3rd, 4th or 5th position is 3 to 65 mole %, and preferably 5 to 60 mole %, and the total quantity of these olefins is 80 mole % or more, and preferably more than 85 mole % in the starting material for preparing the sizing agent.

The mixture of the straight chain olefins can be discriminated clearly from any of branched olefins and α-olefins that are disclosed in U.S. Patent No. 3,102,064.

In the olefins as disclosed in U.S. Patent No. 3,821,069, double bonds exist in the middle part of molecular chains at 6th position (those having 12 carbon atoms) to 12th position (those having 24 carbon atoms) provided that the olefins are straight chain olefins. This fact will be understood from the foregoing general formula. Therefore, these olefins can be discriminated from the olefins of the present invention in which 80 mole % or more of olefins have double bonds at 2nd, 3rd, 4th or 5th position.

Furthermore, in the olefins disclosed in Japanese Preliminary Patent Publication No. 57—154495, double bonds are evenly distributed to every position other than α-position. In the case of olefins having 14 carbon atoms (lower limit) of the present invention, possible positions of double bonds are 1st position (α-position) to 7th position (in the middle of molecular chain) and the total quanitity of 2nd to 5th positions is 80 mole % or more. Accordingly, average quantity of each of 2nd to 5th positions is more than 20% and the total of 1st, 6th and 7th positions is less than 20 mole %. Therefore, the average of 1st, 6th and 7th positions is 6.7%. Assuming that 1st position does no exist, the average of 6th and 7th positions is less than 10%. Therefore, it cannot be said that the double bonds in the starting material of the invention are distributed evenly. Still further, with the increase of carbon atom number, the percentages of 6th or higher positions becomes small, which means that such the case is far from the even distribution of double bonds. Accordingly, the straight chain olefins of the present invention are apparently different from the olefins that are disclosed in Japanese Preliminary Patent Publication No. 57—154495.

As explained above, the mixture of olefins used in the present invention is quite different from the olefins that have been proposed in the conventional art, in view of the distribution of double bonds. Owing to the use of such different olefins, a paper sizing agent having excellent sizing effect as compared with those prepared from the conventional olefin starting materials can be obtained. This characteristic advantage cannot be suggested by any prior art.

The distribution of double bonds in the straight chain olefins can be obtained by $^{13}C$—NMR analysis. According to $^{13}C$—NMR analysis, the chemical shifts are as follows:

| | |
|---|---|
| 1st position C-atom of α-olefin: | 114.2 ppm |
| 2nd position C-atom of 2-olefin (trans): | 124.5 ppm |
| 2nd position C-atom of 2-olefin (cis) | 123.5 ppm |
| 3rd position C-atom of 3-olefin (trans): | 131.9 ppm |
| 3rd position C-atom of 3-olefin (cis): | 131.5 ppm |
| 4th position C-atom of 4-olefin (trans): | 130.15 ppm |

| | |
|---|---|
| 4th position C-atom of 4-olefin (cis): | 129.63 ppm |
| 5th position C-atom of 5-olefin (trans): | 130.37 ppm |
| 5th position C-atom of 5-olefin (cis: | 129.85 ppm |
| Double bond C-atom of other olefin (trans): | 130.44 ppm |
| Double bond C-atom of other olefin (cis): | 129.92 ppm |

The composition of olefins having double bonds at 1st, 2nd, 3rd, 4th, 5th and 6th or higher positions can be determined from the ratio of strengths of these chemical shifts.

The straight chain olefins that are used in the present invention can be prepared without difficulty by isomerizing straight chain α-olefins having corresponding numbers of carbon atoms in the presence of an acid catalyst under appropriate reaction conditions. The straight chain α-olefins as the starting materials of the invention can be easily obtained industrially from polymerization of ethylene by Ziegler process or thermal cracking of wax.

Exemplified as the acid catalyst for use in the isomerization are homogeneous or heterogeneous catalysts of acid minerals such as synthetic silica alumina, synthetic silica magnesia, solid phosphoric acid, acid clay, activated clay, heteropoly-acids such as $H_3PW_{12}O_{40}$, $H_4SiW_{12}O_{40}$ and $H_3PMo_{12}O_{40}$, and $AlCl_3$-electron donor and $BF_3$-electron donor.

The reaction conditions are as follows:

temperatures: 30—250°C,

pressures: normal pressure — 19.6 bar (20 kg/cm$^2$), and

reaction times: 30 minutes — 10 hours.

These conditions can be properly selected according to the kinds of starting α-olefins and catalyst. The type of reaction may be any of batchwise, semi-batchwise and continuous. When continuous reaction is employed, the values of LHSV (liquid hourly space velocity) is preferably set in the range of 0.5 to 20.

When the above straight chain α-olefins are isomerized in the presence of a catalyst, they can be treated with or without an inert medium. Furthermore, it is desirable for maintaining the catalytic activity of the isomerization acid catalyst that the olefins are previously subjected to clay treatment at room temperature.

As described above, the straight chain olefins of the invention can be obtained by isomerizing α-olefins in the presence of an acid catalyst, in which it is necessary that the content of unreacted α-olefins in the reaction product is not more than 20% and preferably 10% or less.

In the isomerization, a small amount of dimer and other heavier olefins of α-olefins are also produced. However, when the reaction product is used for preparing a sizing agent, it can be employed without removing the heavier olefins. From the economical viewpoint, the use without removal is preferable.

The addition of maleic anhydride to the thus prepared straight chain olefin mixture is carried out according to conventional methods.

That is, the reaction can easily proceed heating both the materials to 180—250°C under normal pressure or an elevated pressure in an inert gas. The molar ratio of both the materials is not especially restricted, however, 0.4 to 2 moles of maleic anhydride to 1 mole of the straight chain olefin mixture is generally adopted. After the reaction, the maleic-modified reaction product of the invention can be obtained by removing unreacted maleic anhydride and remaining straight chain olefin mixture by reduced pressure distillation.

In this addition reaction, the main reaction product is an adduct of 1 mole of straight chain olefin with 1 mole of maleic anhydride. However, small quantities of by-products such as an adduct of 1 mole of straight chain olefin with 2 moles of maleic anhydride, 1:2-adduct; and polymer of maleic anhydride are simultaneously produced. These by-products may be either removed or not removed. From economical viewpoint, it is desirable to use the reaction product as a sizing agent without removing the by-products. The reaction product can be further subjected to ordinary hydrogenation process with using a solid catalyst such as palladium or Raney nickel to obtain alkyl succinic anhydride, which product comes also within the scope of the present invention.

When paper pulp is treated with the sizing agent obtained in the above-described process, the sizing agent is homogeneously dispersed in water by a forced mixing apparatus such as a homogeneous mixer, homogenizer or high pressure emulsifier and the obtained dispersion is then added to pulp slurry.

When the sizing agent is dispersed in water, a dispersing agent or protective colloid such as cationized starch, gelatin, polyvinyl alcohol, cationic polyacrylamide or polyethylene imine, or a nonionic emulsifier can be used together. Among the above dispersing agents, the cationized starch, cationic polyacrylamide and polyethylene imine have also the effect as fixing agents to pulp.

The nonionic emulsifiers are exemplified by polyoxyalkylene sorbitan fatty acid ester such as polyoxyethylene sorbitan trioleate; polyoxyalkylene sorbitol fatty acid esters such as polyoxyethylene sorbitol hexaoleate, polyethylene sorbitol laurate and polyoxyethylene sorbitol oleate laurate; polyoxyalkylene alkyl ether such as polyoxyethylene alkyl ether; polyoxyalkylene alkylaryl ether such as polyoxyethylene alkylphenol; polyoxyalkylene monoester and diester such as polyoxyethylene alkyl esters; polyoxyalkylene alkylamine such as polyoxyethylene alklyamine; polyoxyalkylene alkylamide such as polyoxyethylene alkylamide; ester of polyalcohol with fatty acid; and higher alcohol.

In the present invention, the quantity of the foregoing protective colloid used together with the paper

sizing agent, is determined according to the dispersing property of the paper sizing agent in water and the fixing property to pulp of prepared paper sizing medium. The quantity of the protective colloid is generally in the range of 50 to 500%, preferably 100 to 200%, relative to the paper sizing agent.

The quantity of nonionic emulsifier is determined according to the dispersing property of paper sizing agent, and is generally in the range of 3 to 30%, and preferably 5 to 20% relative to the paper sizing agent.

In the case that the above nonionic emulsifier is previously mixed into the paper sizing agent of the present invention and the mixture is then dispersed in water, any forced mixing apparatus such as those used in dispersing with a protective colloid is not necessary. The sizing agent with nonionic emulsifer can be easily dispersed only by using a pulp-mixing aspirator or by passing the mixture through an orifice (these methods are hereinafter referred to as "self-emulsifying method"). Therefore the paper making process can be simplifed.

The quantity of paper sizing agent used in the present invention varies to some extent according to the kind and usages of paper to be produced. The quantity of the paper sizing agent is, however, generally in the range of 0.05 to 5.0% by weight to dry-basis pulp. It is possible to add more than 5.0% by weight of paper sizing agent but the increase of sizing effect can be hardly expected so that the addition of more than 5% is not advantageous in economical viewpoint.

As described above, when the paper sizing agent of this invention is used together with a cationic protective colloid or a nonionic emulsifying agent in neutral or alkaline conditions, the sizing agent can be dispersed well in water. Owing to the use of the specific olefins, the sizing agent produces excellent sizing effect, that is, desired sizing effect can be obtained by using a smaller quantity of the sizing agent as compared with the conventional art. Furthermore, it maintains good sizing effect during lond term preservation, that is, the sizing effect of prepared sizing medium after long term storage is almost the same as the effect that is produced just after it is made up. This fact is more advantageous as compared with the conventional sizing agents.

The paper sizing agent of this invention can be used of course singly, and if desired, it can be used together with known sizing agents by mixing with them in any mixing ratio.

It should be noted also that known pigments and fillers such as clay, talc, titanium oxide, calcium carbonate, calcium sulfate and diatomaceous earth can be used in paper making process together with the sizing agent of the present invention.

The preparation and uses of the sizing agent of the invention will be described in more detail in the following examples. It should be noted, however, that the present invention be limited not by the specific disclosure herein but only by the appended claims.

Example 1

A stainless steel-made continuous reaction tube was fed with 100 ml of synthetic silica alumina and it was maintained at 150°C. n-Hexadecene-1 was fed to this reaction tube at a feed rate of 300 ml/h. According to gas chromatography analysis, the reaction mixture obtained from the outlet of reaction tube contained 7% by weight of heavier components. The heavier components were removed by distillation to obtain n-hexadecenes. The composition of the n-hexadecenes was determined by $^{13}C$—NMR analysis, the result of which is shown in the following Table 1.

Table 1

| Components | Contents |
|---|---|
| n-Hexadecene-1 | 5 mole % |
| n-Hexadecene-2 | 57 mole % |
| n-Hexadecene-3 | 18 mole % |
| n-Hexadecene-4 | 11 mole % |
| n-Hexadecene-5 | 6 mole % |
| O t h e r s | 3 mole % |

The mixture of 450 g of n-hexadecenes and 195 g of maleic anhydride was fed into a stainless steel-made reaction vessel equipped with a stirrer and a thermometer. The atmosphere in the reaction system

was sufficiently replaced with dry nitrogen gas and the above materials were allowed to react for 20 hours at 120°C. After the reaction, unreacted olefins and maleic anhydride were removed by distillation to obtain 450 g of maleic-modified reaction product.

Example 2

Using $C_{16}$—$C_{18}$ α-olefin in place of n-hexadecene-1 of Example 1, isomerization was carried out. The α-olefin was obtained by Ziegler method and contained 56% by weight of $C_{16}$ and 44% by weight of $C_{18}$. The heavier component in the obtained isomerization product was 2% by weight. The composition of the reaction product besides the heavier component was determined by $^{13}C$—NMR analysis, the result of which is shown in the following Table 2.

Table 2

| Components | Contents |
|---|---|
| 1-Olefin | 7 mole % |
| 2-Olefin | 46 mole % |
| 3-Olefin | 23 mole % |
| 4-Olefin | 13 mole % |
| 5-Olefin | 8 mole % |
| Others | 3 mole % |

480 g of the thus obtained straight chain olefin mixture (2% by weight of heavier component was contained) and 195 g of maleic anhydride was fed into a stainless steel-made reaction vessel equipped with a stirrer and a thermometer. The atmosphere in the reaction system was sufficiently replaced with dry nitrogen gas and the above materials were allowed to react for 30 hours at 190°C. After the reaction, unreacted olefins and maleic anhydride were removed by distillation to obtain 435 g of maleic-modified reaction product.

Example 3

A stainless steel-made continuous reaction tube was fed with 100 ml of 12-tungstosilicic acid carried on silica gel and it was maintained at 100°C. n-Octadecene-1 was fed to this reaction tube at a feed rate of 300 ml/h. The reaction mixture obtained from the outlet of reaction tube contained no heavier component. The composition of the reaction product was determined by $^{13}C$—NMR analysis, the result of which is shown in the following Table 3.

Table 3

| Components | Contents |
|---|---|
| n-Octadecene-1 | 3 mole % |
| n-Octadecene-2 | 63 mole % |
| n-Octadecene-3 | 14 mole % |
| n-Octadecene-4 | 12 mole % |
| n-Octadecene-5 | 5 mole % |
| Others | 3 mole % |

Under the same conditions as those in Example 2, 500 g of this olefin mixture and 190 g of maleic anhydride were reacted to obtain 450 g of maleic-modified reaction product.

Example 4

Isomerization was carried out in the like manner as Example 1 using $C_{18}$—$C_{20}$ α-olefin in place of n-hexadecene-1. The α-olefine was obtained by decomposition of wax and contained 24% by weight of $C_{18}$, 39% by weight of $C_{19}$ and 37% by weight of $C_{20}$. The α-olefin was subjected to clay treatment at room temperature before it was fed to synthetic silica alumina. The obtained isomerization product contained no heavier component. The composition of the reaction product was determined by $^{13}C$—NMR analysis, the result of which is shown in the following Table 4.

Table 4

| Components | Contents |
|---|---|
| 1-Olefin | 5 mole % |
| 2-Olefin | 49 mole % |
| 3-Olefin | 28 mole % |
| 4-Olefin | 13 mole % |
| 5-Olefin | 4 mole % |
| Others | 1 mole % |

Under the same conditions as those in Example 2, 600 g of this olefin mixture and 190 g of maleic anhydride were reacted to obtain 474 g of maleic-modified reaction product.

Comparative Example 1

In the like manner as the addition reaction in Example 1, 300 g of n-octadecene-9 and 115 g of maleic anhydride were reacted to obtain 248 g of maleic-modified reaction product.

Comparative Example 2

A mixture of n-paraffins of 15 to 18 in carbon atom number and hydrogen gas were subjected to

dehydrogenation by feeding them into a stainless steel-made reaction vessel containing 30 ml of catalyst, $Pt \cdot Li_2O \cdot Al_2O_3$. The reaction conditions were: reaction temperature: 470°C, feeding rate: 15 ml/min, and molar ratio of $H_2$/n-paraffin:8.0. The olefin content of the effluent from the outlet of reaction vessel was about 12% by weight. This reaction mixture was passed through a molecular sieve to obtain n-olefins. The distribution of carbon numbers of this n-olefins was $C_{18}$: 5% by weight, $C_{16}$: 37% by weight, $C_{17}$: 38% by weight and $C_{18}$: 20% by weight. According to $^{13}C$—NMR analysis, the composition of the olefins was shown in the following Table 5.

Table 5

| Components | Contents |
|---|---|
| 1-Olefin | 2 mole % |
| 2-Olefin | 13 mole % |
| 3-Olefin | 15 mole % |
| 4-Olefin | 18 mole % |
| 5-Olefin | 17 mole % |
| Others | 35 mole % |

In the same manner as Example 1, 95 g of maleic anhydride was added to 240 g of the olefins to obtain 220 g of maleic-modified reaction product.

Comparative Example 3

Example 3 was traced by using n-tridecene-1 in place of n-octadecene-1 to obtain maleic-modified reaction product.

Comparative Example 4

In the same manner as the addition reaction in Example 1, 130 g of maleic anhydride was reacted with 300 g of oligomers having 15 to 18 carbon atoms ($C_{25}$: 63%, $C_{26}$: 13%, $C_{27}$: 7% and $C_{18}$: 17% by weight) which were obtained by oligomerization of propylene, and 213 g of maleic-modified reaction product was obtained.

Evaluation Tests as Sizing Agents

With regard to the maleic-modified reaction products obtained in the foregoing Examples 1 to 4 and Comparative Examples 1 to 4, effects as sizing agents were evaluated through the following method of use.

Use Example 1

To 1 g of maleic-modified reaction product were added 20 g of 10% cationized starch solution and 79 g of water, and the mixture was emulsified by a homogenizer. The obtained emulsion was added to 1% pulp slurry (L—BKP 450 ml c.s.f.), in which the concentrations of maleic-modified reaction product were made 0.2% and 0.5% by weight (to dry pulp). Then, paper of 60 ± 1 g/m$^2$ in basis weight was made by using a TAPPI standard machine, in which 20% by weight (to dry pulp) of $CaCO_3$ was used as a filler.

After that, the obtained wet paper was dehydrated by a press and dried for 1 minute at 100°C. The moisture of paper was then adjusted by placing it in an atmosphere of 65% relative humidity for 24 hours. The properties of paper were determined by Stöchigt method, the results of which are shown in the following Table 6.

It will be understood that the sizing degrees of the sizing agents of the present invention are higher than those of the sizing agents in Comparative Examples.

Table 6

| Test Item | Stöchigt Sizing Degree (s) | |
|---|---|---|
| Conc. of Sizing Agent | 0.2% | 0.5% |
| Example 1 | 10 | 23 |
| Example 2 | 12 | 27 |
| Example 3 | 9 | 25 |
| Example 4 | 12 | 26 |
| Comparative Example 1 | 7 | 20 |
| Comparative Example 2 | 8 | 20 |
| Comparative Example 3 | 0 | 7 |
| Comparative Example 4 | 6 | 19 |

Use Example 2

To 10 g of maleic-modified reaction product was added, as an emulsifier, 1 g of polyethylene glycol nonylphenyl ether (trademark: Nonipole 160 made by Sanyo Kasei Kogyo Kabushiki Kaisha) and the mixture was well stirred together. Then, 99 g of water were added to 1 g of this mixture and this mixture was emulsified by stirring for 10 seconds with a propeller agitator to obtain a sizing medium.

0.3% by weight (based on dry pulp) of polyamide polyamine resin (trademark: AF—100 made by Arakawa Kagaku Kogyo Kabushiki Kaisha) as a fixing agent was added to 1% slurry of pulp and the above sizing medium was then added to this mixture in a quantity of 0.5% by weight of maleic-modified reaction product to dry pulp. In the like manner as Use Example 1, sizing degrees were determined. Furthermore, the sizing degrees of the dilute sizing mediums at 5 hours after preparation were also determined. These results are shown in the following Table 7.

Table 7

| Test Item | Stöchigt Sizing Degree (s) | |
|---|---|---|
| Time Tested | Just after Emulsifying | 5 h after Emulsifying |
| Example 1 | 25 | 8 |
| Example 2 | 28 | 14 |
| Example 3 | 24 | 10 |
| Example 4 | 28 | 15 |
| Comparative Example 1 | 22 | 0 |
| Comparative Example 2 | 22 | 4 |

As will be understood from the results shown in Table 7, the self-emulsifying type sizing agent of the present invention is superior in the sizing degrees of just after preparation as compared with those in Comparative Examples. Furthermore, the lowering in sizing degrees of sizing agent of the invention at 5 hours after emulsifying is quite small.

**Claims for the Contracting States: BE CH DE FR GB IT LI LU NL SE**

1. A paper sizing agent which comprises a reaction product which is obtained by adding maleic anhydride to a mixture of straight chain olefins having 14 to 36 carbon atoms, and/or the hydrogenation product of said reaction product, characterized in that said mixture of straight chain olefins comprises 3 to 65 mole %, respectively, of olefins having a double bond at the 2nd, 3rd, 4th or 5th position, and the total quantity of olefins having internal double bonds being not less than 80 mole %.

2. The paper sizing agent of claim 1, wherein said mixture of straight chain olefins is prepared by isomerizing straight chain α-olefins having the same number of carbon atoms in the presence of an acid catalyst.

3. The paper sizing agent of claim 2, wherein said straight chain α-olefins having the same number of carbon atoms is subjected to clay treatment previously to said isomerization.

4. The paper sizing agent of any of the claims 1 to 3, wherein said sizing agent contains a dispersing agent.

5. The paper sizing agent of claim 4, wherein said dispersing agent is at least one member selected from the group consisting of cationized starch, gelatin, polyvinyl alcohol, cationic polyacrylamide and polyethylene imine.

6. The paper sizing agent of any of the claims 1 to 5, wherein said sizing agent contains an emulsifying agent.

7. The paper sizing agent of claim 6, wherein said emulsifying agent is at least one member selected from the group consisting of polyoxyalkylene sorbitol fatty acid ester, polyoxyalkylene sorbitan fatty acid ester, polyoxyalkylene alkyl ether, polyoxyalkylene alkylaryl ether, polyoxyalkylene monoester, and polyoxyalkylene diester.

**Claims for the Contracting State: AT**

1. A process for producing a paper sizing agent which comprises adding maleic anhydride to a mixture of straight chain olefins having 14 to 36 carbon atoms, characterized in that said mixture of straight chain olefins comprises 3 to 65 mole %, respectively, of olefins having a double bond at the 2nd, 3rd, 4th or 5th position, that the total quantity of these olefins is not less than 80 mole % and that the obtained reaction product is optionally hydrogenated.

2. Process according to claim 1, wherein said mixture of straight chain olefins is prepared by isomerizing straight chain α-olefins having carbon atoms of the same numbers in the presence of an acid catalyst.

3. Process according to claim 2, wherein said straight chain α-olefin having carbon atoms of the same numbers is subjected to clay treatment previously to said isomerization.

4. Process according to any of the claims 1 to 3, wherein the addition reaction is carried out by heating maleic anhydride together with a mixture of straight chain olefins to a temperature of 180 to 250°C under normal pressure or under elevated pressure in an inert gas atmosphere.

5. Paper sizing composition comprising a dispersion in water of a reaction product and/or hydrogenation product of said reaction product, which reaction product is obtained by adding maleic anhydride to a mixture of straight chain olefins having 14 to 36 carbon atoms, characterized in that said mixture of straight chain olefins comprises 3 to 65 mole %, respectively, of olefins having a double bond at the 2nd, 3rd, 4th or 5th position and that the total quantity of these olefins is not less than 80 mole %.

6. Paper sizing composition according to claim 5, which additionally comprises a dispersing agent or a protective colloid.

**Patentansprüche für die Vertragsstaaten: BE CH DE FR GB IT LI LU NL SE**

1. Papierleimungsmittel, enthaltend ein Reaktionsprodukt, das durch Addition von Maleinsäureanhydrid an ein Gemisch von geradekettigen Olefinen mit 14 bis 36 Kohlenstoffatomen erhalten wurde und/oder das Hydrierungsprodukt dieses Reaktionsprodukts, dadurch gekennzeichnet, daß das Gemisch aus geradekettigen Olefinen jeweils 3 bis 65 Mole.-% an Olefinen enthält, die eine Doppelbindung in 2-Stellung, 3-Stellung, 4-Stellung oder 5-Stellung haben, und die Gesamtmenge an Olefinen mit innenständigen Doppelbindungen nicht weniger als 80 Mol.-% beträgt.

2. Papierleimungsmittel nach Anspruch 1, wobei das Gemisch aus geradekettigen Olefinen durch Isomerisieren von geradkettigen α-Olefinen mit der gleichen Anzahl an Kohlenstoffatomen in Gegenwert eines Säure-Katalysators hergestellt wurde.

3. Papierleimungsmittel nach Anspruch 2, wobei die geradekettigen α-Olefine mit der gleichen Anzahl

an Kohlenstoffatomen vor der Isomerisierung einer Behandlung mit Ton unterworfen wurden.

4. Papierleimungsmittel nach einem der Ansprüche 1 bis 3, wobei das Leimungsmittel ein Dispergiermittel enthält.

5. Papierleimungsmittel nach Anspruch 4, in dem das Dispergiermittel mindestens ein Mitglied aus der aus kationisierter Stärke, Gelatine, Polyvinylalkohol, kationischem Polyacrylamid und Polyethylenimid bestehenden Gruppe ist.

6. Papierleimungsmittel nach einem der Ansprüche 1 bis 5, wobei das Leimungsmittel ein Emulgiermittel enthält.

7. Papierleimungsmittel nach Anspruch 6, in dem das Emulgiermittel mindestens ein Mitglied aus der aus Polyoxyalkylen-sorbitol-fettsäureester, Polyoxyethylen-sorbitan-fettsäureester, Polyoxyalkylenalkylether, Polyoxyalkylen-alkylarylether, Polyoxyalkylen-monoester und Polyoxyalkylen-diester bestehenden Gruppe ist.

**Patentansprüche für den Vertragsstaat: AT**

1. Verfahren zur Herstellung eines Papierleimungsmittels, bei dem Maleinsäureanhydrid an ein Gemisch aus geradekettigen Olefinen mit 14 bis 36 Kohlenstoffatomen addiert wird, dadurch gekennzeichnet, daß das Gemisch aus geradekettigen Olefinen jeweils 3 bis 65 Mol.-% an Olefinen enthält, die ein Doppelbindung in 2-Stellung, 3-Stellung, 4-Stellung oder 5-Stellung aufweisen, daß die Gesamtmenge dieser Olefine nicht weniger als 80 Mol.-% beträgt und daß das erhaltene Reaktionsprodukt gegebenenfalls hydriert wird.

2. Verfahren nach Anspruch 1, bei dem das Gemisch aus geradekettigen Olefinen durch Isomerisierung von geradekettigen α-Olefinen mit der gleichen Anzahl der Kohlenstoffatome in Gegenwart eines Säurekatalysators hergestellt wurde.

3. Verfahren nach Anspruch 2, bei dem die geradekettigen α-Olefine mit der gleichen Anzahl der Kohlenstoffatome vor der Isomerisierung einer Ton-Behandlung unterworfen wurden.

4. Verfahren nach einem der Ansprüche 1 bis 3, bei dem die Additionsreaktion durch Erhitzen von Maleinsäureanhydrid zusammen mit einem Gemisch aus geradekettigen Olefinen auf eine Temperatur von 180 bis 250°C unter Normaldruck oder unter erhöhtem Druck in einer inerten Gasatmosphäre durchgeführt wird.

5. Papierleimungsmittel-Zusammensetzung, enthaltend eine wässerige Dispersion eines Reaktionsprodukts und/oder Hydrierungsprodukts dieses Reaktionsprodukts, wobei das Reaktionsprodukt durch Addition von Maleinsäureanhydrid an ein Gemisch aus geradekettigen Olefinen mit 14 bis 36 Kohlenstoffatomen erhalten wird, dadurch gekennzeichnet, daß das Gemisch aus geradekettigen Olefinen jeweils 3 bis 65 Mol.-% an Olefin enthält, die eine Doppelbindung in 2-Stellung, 3-Stellung, 4-Stellung oder 5-Stellung aufweisen und daß die Gesamtmenge dieser Olefine nicht weniger als 80 Mol.-% beträgt.

6. Papierleimungsmittel-Zusammensetzung nach Anspruch 5, die zusätzlich ein Dispergiermittel oder ein Schutzkolloid enthält.

**Revendications pour les Etat contractants: BE CH DE FR GB IT LI LU NL SE**

1. Agent d'encollage pour papier qui comprend un produit de réaction obtenu par addition d'anhydride maléique à un mélange d'oléfines à chaîne linéaire ayant 14 à 36 atomes de carbone, et/ou le produit d'hydrogénation de ce produit de réaction, caractérisé en ce que ce mélange d'oléfines à chaîne linéaire comprend de 3 à 65 moles % d'oléfines ayant respectivement une double liaison en seconde, troisième, quatrième ou cinquième position, et en ce que la quantité totale d'oléfines ayant des doubles liaisons internes n'est pas inférieure à 80 moles %.

2. Agent d'encollage pour papier selon la revendication 1, dans lequel le mélange d'oléfines à chaîne linéaire est préparé par isomérisation d'α-oléfines à chaîne linéaire ayant le même nombre d'atomes de carbone, en présence d'un catalyseur acide.

3. Agent d'encollage pour papier selon la revendication 2, dans lequel les α-oléfines à chaîne linéaire, ayant le même nombre d'atomes de carbone, sont soumises, préalablement à l'isomérisation, à un traitement à l'argile.

4. Agent d'encollage pour papier selon une des revendications 1 à 3, qui contient un agent dispersant.

5. Agent d'encollage pour papier selon la revendication 4, dans lequel l'agent de dispersion est constitué par au moins un corpschoisi parmi les produits: amidon cationisé, gélatine, alcool polyvinylique, polyacrylamide cationique et polyéthylène imine.

6. Agent d'encollage pour papier selon une des revendications 1 à 5, qui renferme un agent émulsionnant.

7. Agent d'encollage pour papier selon la revendication 6, dont l'agent émulsionnant est choisi parmi ceux qui comprennent: ester d'acide gras de polyoxyalkylène sorbitol, ester d'acide gras de polyoxyalkylène sorbitane, éther polyoxyalkylène alkylique, éther polyoxyalkylène alkylarylique, monoester de polyoxyalkylène et diester de polyoxyalkylène.

**Revendications pour l'Etat contractant: AT**

1. Procédé de préparation d'un agent d'encoullage pour papier, dans lequel de l'anhydride maléique est additioné à un mélange d'olefines à chaîne linéaire ayant 14 à 36 atomes de carbone, caractérisé en ce que ce mélange d'oléfines à chaîne linéaire comprend de 3 à 65 moles % d'oléfines ayant respectivement une double liaison en seconde, troisième, quatrième ou cinquième position, et en ce que la quantité totale de ces oléfines n'est pas inférieure à 80 moles % et en ce que le produit de réaction obtenu est facultativement hydrogéné

2. Procédé selon la revendication 1, dans lequel le mélange d'oléfines à chaîne linéaire est préparé par isomérisation d'α-oléfines à chaîne linéaire ayant le même nombre d'atomes de carbone, en présence d'un catalyseur acide.

3. Procédé selon la revendication 2, dans lequel les α-oléfines à chaîne linéaire, ayant le même nombre d'atomes de carbone, sont soumises, préalablement à l'isomérisation, à un traitement à l'argile.

4. Procédé selon une des revendications 1 à 3, dans lequel la réaction d'addition s'effectue par chauffage d'anhydride maléique, ensemble avec un mélange d'oléfines à chaîne linéaire, à une température de 180 à 250°C sous pression normale ou à pression élevée dans une atmosphère de gaz inerte.

5. Composition d'agent d'encollage pour papier qui comprend une dispersion aqueuse d'un produit de réaction et/ou d'un produit d'hydrogénation de ce produit de réaction, le produit de réaction étant obtenu par addition d'anhydride maléique à un mélange d'oléfines à chaîne linéaire ayant 14 à 36 atomes de carbone, caractérisé en ce que le mélange d'oléfines à chaîne linéaire comprend de 3 à 65 moles % d'oléfines ayant respectivement une double liaison en seconde, troisième, quatrième ou cinquième position, et en ce que la quantité totale de ces oléfines n'est pas inférieure à 80 moles %.

6. Composition d'agent d'encollage pour papier selon la revendication 5, qui additionallement contient un agent dispersant ou un colloide protecteur.